# EUROPEAN PATENT APPLICATION

(11) **EP 2 292 785 A1**
(43) Date of publication of application: **09.03.2011**
(21) Application number: 10251526.9
(22) Date of filing: 27.08.2010
(51) Int. Cl.: C12Q 1/00, G01N 27/00, G01N 33/52

(54) **Multi-analyte test strip with shared counter/reference electrode and inline electrode configuration**

(30) Priority: 31.08.2009 US 551157
(71) Applicant: Lifescan Scotland Limited, Inverness-Shire IV2 3ED (GB)
(72) Inventor: Moffat, James, Inverness Inverness-shire, IV3 8QU (GB); Macleod, Kathryn, Inverness Inverness-shire, IV3 5RB (GB); Leach, Christopher Philip, Inverness Inverness-shire, IV2 3DP (GB); Macfie, Gavin, Inverness Inverness-shire, IV2 5ES (GB); Lillie, Geoffrey, Inverness Inverness-shire, IV3 5JW (GB); Cardosi, Marco F., Croy Inverness-shire, IV23 5FP (GB); Saini, Selwayan, Culbokie Dingwall, IV7 8JS (GB)
(74) Representative: Tunstall, Christopher Stephen

(57) **Abstract**

A multi-analyte test strip includes a first insulating layer and an electrically conductive layer disposed on the first insulating layer. The electrically conductive layer has a first working electrode with a first analyte contact pad, a shared counter/reference electrode with a counter/reference electrode contact pad, and a second working electrode with a second analyte contact pad. The multi-analyte test strip also includes a second insulating layer disposed above the first insulating layer and a patterned spacer layer positioned between the first insulating layer and the first electrically conductive layer with the patterned spacer layer defining a bodily fluid sample-receiving chamber that overlies the first working electrode, the shared counter/reference electrode and the second working electrode. The multi-analyte test strip further includes a mediator reagent layer disposed over the first working electrode, the shared counter/reference electrode and the second working electrode; a first analyte reagent layer disposed over the first working electrode and mediator reagent layer; and a second analyte reagent layer disposed over the second working electrode and mediator reagent layer. Furthermore, the first analyte electrode, shared counter/reference electrode and second analyte electrode of the multi-analyte test strip are disposed on the first insulating layer in a planar inline configuration.

## Description

### BACKGROUND OF THE INVENTION

Field of the Invention

The present invention relates, in general, to medical devices and, in particular, to analyte test strips, test meters and related methods.

Description of Related Art

The determination (e.g., detection and/or concentration measurement) of an analyte in a fluid sample is of particular interest in the medical field. For example, it can be desirable to determine glucose, ketones, cholesterol, acetaminophen and/or HbA1c concentrations in a sample of a bodily fluid such as urine, blood or interstitial fluid. Such determinations can be achieved using analyte test strips, based on, for example, photometric or electrochemical techniques, along with an associated test meter.

Typical electrochemical-based analyte test strips employ a working electrode along with an associated counter/reference electrode and enzymatic reagent to facilitate an electrochemical reaction with a single analyte of interest and, thereby, determine the concentration of that single analyte. For example, an electrochemical-based analyte test strip for the determination of glucose concentration in a blood sample can employ an enzymatic reagent that includes the enzyme glucose oxidase and the mediator ferricyanide. Such conventional analyte test strips are described in, for example, U.S. Patent No.s 5,708,247; 5,951,836; 6,241,862; and 6,284,125; each of which is hereby incorporated in full by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings, in which like numerals indicate like elements, of which:
FIG. 1 is a simplified perspective, exploded depiction of a multi-analyte test strip according to an embodiment of the present invention;
FIG. 2 is a simplified top view of the conductive layer (also referred to as the "inline electrode layer") of the multi-analyte test strip of FIG. 1;
FIG. 3 is a simplified top view of the inline electrode layer and patterned spacer layer of the multi-analyte test strip of FIG. 1 (with dashed lines indicating portions of the inline electrode layer that are blocked from view by the patterned spacer layer);
FIG. 4 is a simplified top view of the inline electrode layer, patterned spacer layer and mediator reagent layer of the multi-analyte test strip of FIG. 1 (with dashed lines indicating portions of the inline electrode layer and patterned spacer layer that are blocked from view by the mediator reagent layer);
FIG. 5 is a simplified top view of the inline electrode layer, patterned spacer layer, mediator reagent layer and first analyte reagent layer of the multi-analyte test strip of FIG. 1;
FIG. 6 is a simplified top view of the inline electrode layer, patterned spacer layer, mediator reagent layer, first analyte reagent layer and second analyte reagent layer of the multi-analyte test strip of FIG. 1;
FIG. 7 is simplified depiction of the conductive layer of a multi-analyte test strip according to an embodiment of the present invention in use with a test meter also according to an embodiment of the present invention; and
FIG. 8 is a flow diagram depicting stages in a process for determining multiple analytes in a single bodily fluid sample according to an embodiment of the present invention.

### DETAILED DESCRIPTION OF ILLUSTRATIVE EMBODIMENTS

The following detailed description should be read with reference to the drawings, in which like elements in different drawings are identically numbered. The drawings, which are not necessarily to scale, depict exemplary embodiments for the purpose of explanation only and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

Multi-analyte test strips according to embodiments of the present invention include a first insulating layer and an electrically conductive layer disposed on the first insulating layer. The electrically conductive layer has a first working electrode with a first analyte contact pad, a shared counter/reference electrode with a counter/reference electrode contact pad, and a second working electrode with a second analyte contact pad.

The multi-analyte test strips also include a second insulating layer disposed above the first insulating layer and a patterned spacer layer positioned between the first insulating layer and the first electrically conductive layer with the patterned spacer layer defining a bodily fluid sample-receiving chamber that overlies the first working electrode, the shared counter/reference electrode and the second working electrode.

The multi-analyte test strips further include (i) at least one mediator reagent layer disposed over the first working electrode, the shared counter/reference electrode and the second working electrode; (ii) a first analyte reagent layer disposed over the first working electrode and mediator reagent layer; and (iii) a second analyte reagent layer disposed over the second working electrode and mediator reagent layer. Furthermore, the first analyte electrode, shared counter/reference electrode and second analyte electrode of the multi-analyte test strip are disposed on the first insulating layer in a planar inline configuration.

Multi-analyte test strips according to the present invention are beneficial in that a plurality of non-identical analytes (e.g., the analyte glucose and the ketone analyte 3-hydroxybutyrate) can be determined in a single bodily fluid sample (such as a single whole blood sample). In addition, since the multi-analyte test strips include a shared counter/reference electrode (i.e., a counter/reference electrode that is used during the determination of both the first analyte and the second analyte), the multi-analyte test strips, and their bodily fluid sample receiving chamber, are beneficially small in size. Moreover, the use of an inline configuration for the first analyte electrode, second analyte electrode and shared counter/reference electrode enables the multi-analyte test strips to employ a straight sample chamber with straight bodily fluid sample flow, thus simplifying manufacturing and use of the multi-analyte test strip.

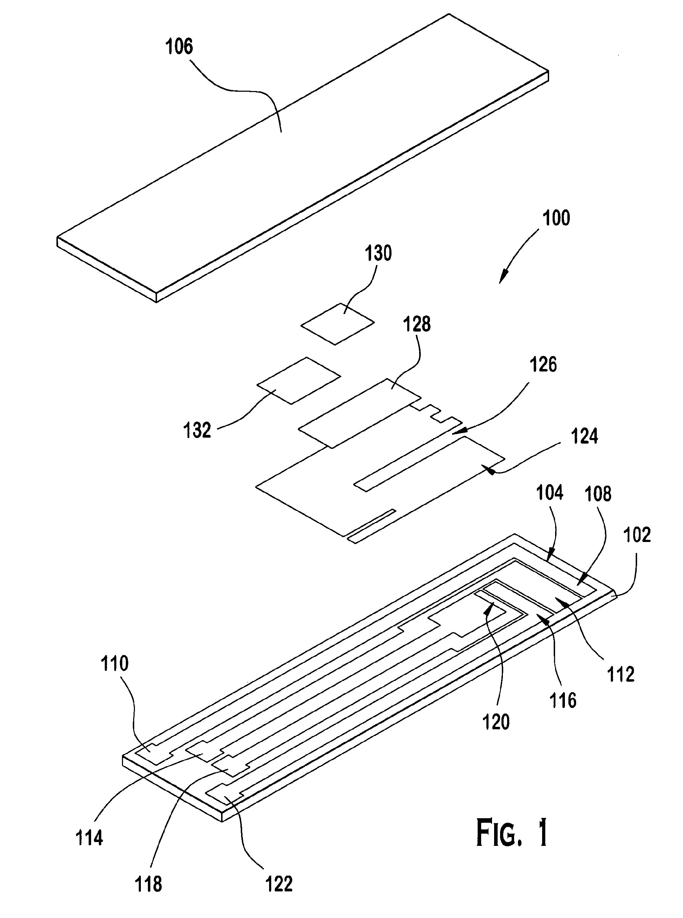

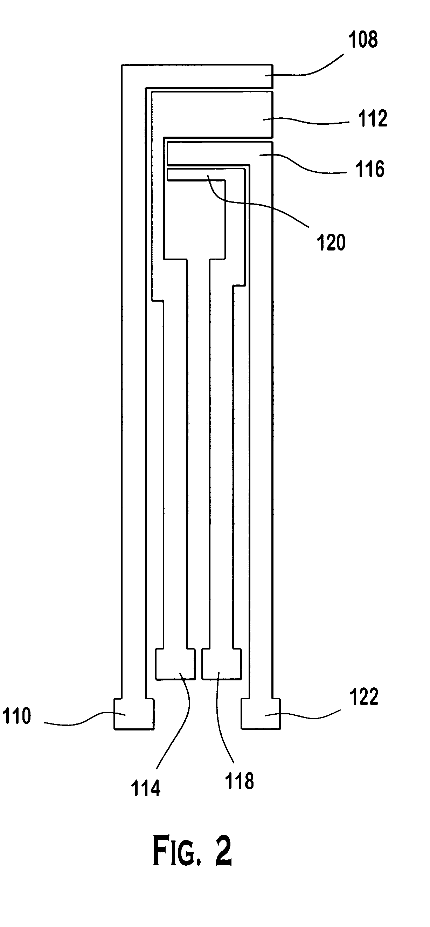

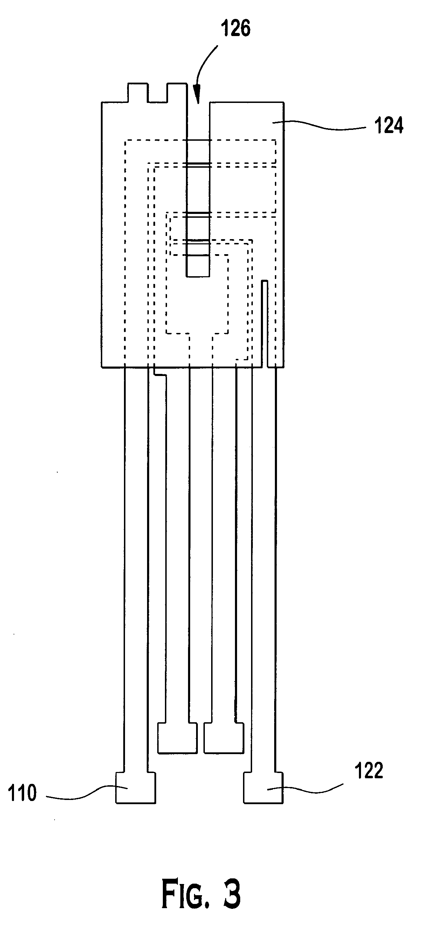

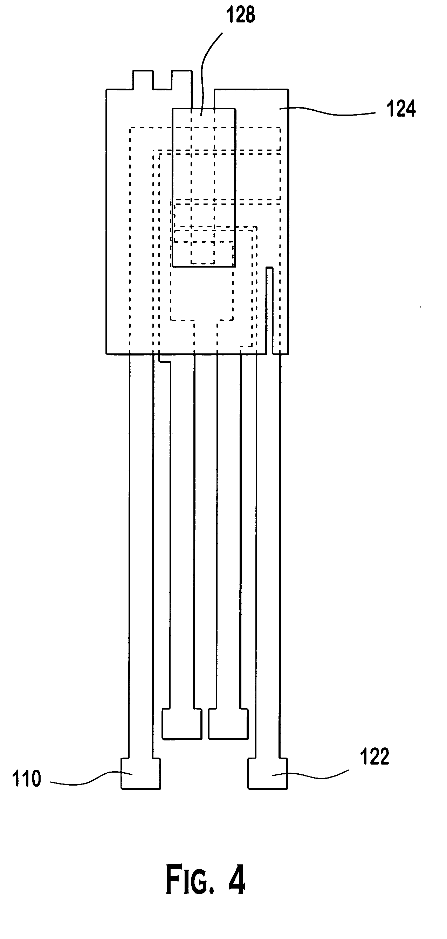

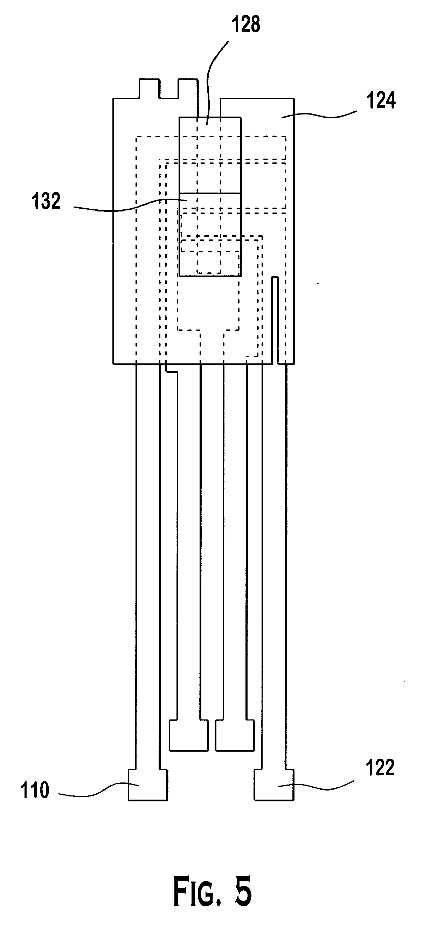

FIG. 1 is a simplified perspective, exploded depiction of a multi-analyte test strip 100 according to an embodiment of the present invention. FIGs. 2 through 6 are simplified top views of various layers of multi-analyte test strip 100.

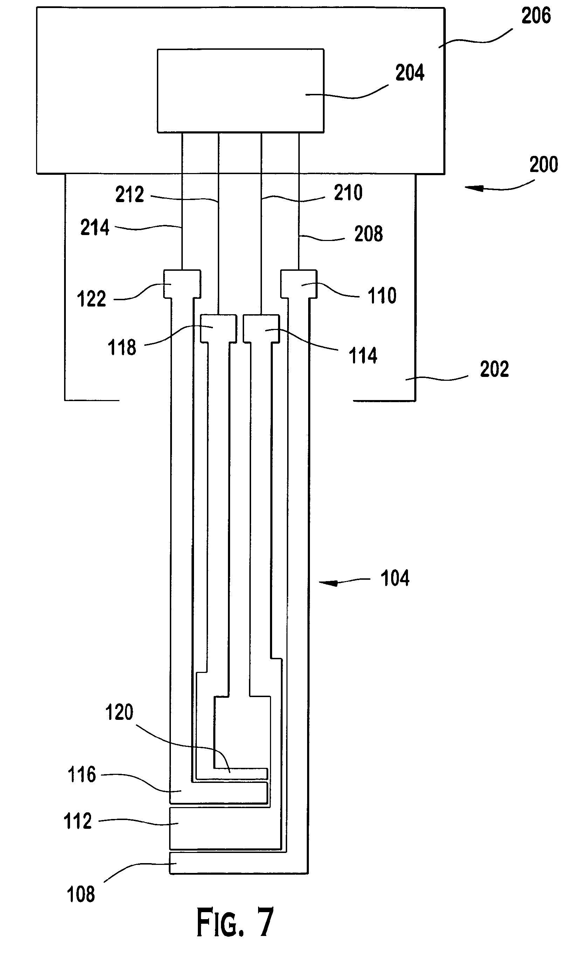

Referring to FIGs. 1-6, multi-analyte test strip 100 is configured for use with a test meter (described further herein, for example with respect to the embodiment of FIG. 7). Multi-analyte test strip 100 includes a first insulating layer 102, with first electrically conductive layer 104 disposed thereon, and a second insulating layer 106. Second insulating layer 106 is disposed above first insulating layer 102.

First electrically conductive layer 104 includes a first working electrode 108 with a first analyte contact pad 110, a shared counter/reference electrode 112 with a counter/reference electrode contact pad 114, as well as a second working electrode 116 with a second analyte contact pad 118, and a fill detect electrode 120 with a fill detect contact pad 122. Once apprised of the present disclosure, one skilled in the art will recognize that a fill detect electrode is optional in some embodiments of multi-analyte test strips according to the present invention. First analyte contact pad 110, counter/reference electrode contact pad 114, second analyte contact pad 118 and fill detect contact pad 122 are configured for contact with an electrical connector pins of a test meter. Moreover, the first analyte electrode, shared counter/reference electrode, second analyte electrode and fill detect electrode are disposed on the first insulating layer in a planar inline configuration.

Multi-analyte test strip 100 also includes a patterned spacer layer 124 positioned between second insulating layer 106 and first electrically conductive layer 104. Patterned spacer layer 124 defines a bodily fluid sample-receiving chamber 126 therein that overlies the first working electrode, the shared counter/reference electrode, the second working electrode, and the fill detect electrode.

Multi-analyte test strip 100 also includes a mediator reagent layer 128 disposed over at least a portion of the first working electrode, at least a portion of the shared counter/reference electrode, at least a portion of the second working electrode, and at least a portion of the fill detect electrode. Multi-analyte test strip 100 also includes a first analyte reagent layer 130 disposed at least over a portion of the first working electrode and mediator reagent layer 128 and a second analyte reagent layer 132 disposed at least over a portion of the second working electrode and mediator reagent layer 128.

Although the embodiment of FIGs. 1-6 includes a single mediator reagent layer that is common to the first working electrode, shared counter/reference electrode and second working electrode, once apprised of the present invention one skilled in the art will recognize that embodiments of the present invention can include a plurality of mediator reagent layers. For example, an embodiment can have a first mediator reagent layer that includes reagents (including mediators) suitable for the determination of a first analyte and be disposed over the first working electrode but not over the second working electrode, and a second mediator reagent layer that includes reagents (including mediators) suitable for the determination of a second analyte that is disposed over the second working electrode but not over the first working electrode. Given that the first and second mediator reagent layers contain different mediators, only one of the first and second mediator reagent layers need be disposed, for example, over the shared counter/reference electrode, thus avoiding chemical incompatibility of the mediators. In addition the stacking order of the at least one mediator reagent layer and the first and second analyte reagent layers can be reversed from that of FIGs. 1-6 (in other words, the mediator reagent layer can be disposed above the first and second analyte reagent layers rather than below, but otherwise aligned as in FIGs. 1-6).

First insulating layer 102 and second insulating layer 106 can be formed, for example, of a suitable plastic (e.g., PET, PETG, polyimide, polycarbonate, polystyrene), silicon, ceramic, or glass material. For example, the first and second insulating layers can be formed from a 7 mil polyester substrate.

In the embodiment of FIGs. 1-6, first working electrode 108 and shared counter/reference electrode 112, along with mediator reagent layer 128 and first analyte reagent layer 130, are configured to electrochemically determine a first analyte concentration in a bodily fluid sample (such as glucose in a whole blood sample) using any suitable electrochemical-based technique known to one skilled in the art. Furthermore, second working electrode 116 and shared counter/reference electrode 112, along with mediator reagent layer 128 and second analyte reagent layer 132, are configured to electrochemically determine a second analyte concentration in the same bodily fluid sample (such as the ketone 3-hydroxybutyrate) in a whole blood sample. Fill detect electrode 120 is configured for the detection (via any suitable technique known to one of skill in the art) of sufficient filling of bodily fluid sample-receiving chamber 126 by a bodily fluid sample.

It should be noted that shared counter/reference electrode 112 is configured to support the operable current density from both the first and the second working electrodes. It should also be noted that as a result of the chemical composition of the mediator reagent layer and/or first analyte reagent layer and/or second analyte reagent layer, the shared counter/reference electrode of multi-analyte test strips according to the present invention is coated with a redox couple that undergoes reduction when an oxidation process is occurring at the first and second working electrodes.

First electrically conductive layer 104 can be formed of any suitable conductive material such as, for example, gold, palladium, carbon, silver, platinum, tin oxide, iridium, indium, or combinations thereof (e.g., indium doped tin oxide). Moreover, any suitable technique can be employed to form first electrically conductive layer 104 including, for example, sputtering, evaporation, electro-less plating, screen-printing, contact printing, or gravure printing. For example, first electrically conductive layer 104 can be a sputtered palladium layer.

Patterned spacer layer 124 serves to bind together first insulating layer 102 (with first electrically conductive layer 104 thereon) and second insulating layer 106. Patterned spacer layer 124 can be, for example, a double-sided pressure sensitive adhesive layer, a heat activated adhesive layer, or a thermo-setting adhesive plastic layer. Patterned spacer layer 124 can have, for example, a thickness in the range of from about 1 micron to about 500 microns, preferably between about 10 microns and about 400 microns, and more preferably between about 40 microns and about 200 microns.

First analyte reagent layer 130 can be any suitable mixture of reagents that, along with mediator reagent layer 128, can selectively react with a first analyte, such as, for example glucose, in a bodily fluid sample to form an electroactive species, which can then be quantitatively measured at the first analyte electrode of multi-analyte test strips according to embodiments of the present invention. Therefore, first analyte reagent layer 130 can include at least an enzyme (with mediator reagent layer 128 including a suitable mediator). Examples of suitable mediators include, for example, ferricyanide, ferrocene, ferrocene derivatives, osmium bipyridyl complexes, and quinone derivatives. Examples of suitable enzymes include glucose oxidase, glucose dehydrogenase (GDH) using a pyrroloquinoline quinone (PQQ) co-factor, GDH using a nicotinamide adenine dinucleotide (NAD) co-factor, and GDH using a flavin adenine dinucleotide (FAD) co-factor. First analyte reagent layer 130 can be applied using any suitable technique.

Second analyte reagent layer 132 can be any suitable mixture of reagents that, along with mediator reagent layer 128, can selectively react with a second analyte such as, for example the ketone 3-hydroxybutyrate, in a bodily fluid sample to form an electroactive species, which can then be quantitatively measured at the second analyte electrode of multi-analyte test strips according to embodiments of the present invention. Therefore, second analyte reagent layer 132 can include at least an enzyme (with mediator reagent layer 128 including a mediator). Second analyte reagent layer 132 can be applied using any suitable technique. It should be noted that the first and second analytes are dissimilar. In other words, the first and second analytes are not the same chemical species. Therefore, two different analytes are determined by multi-analyte test strips according to the present invention.

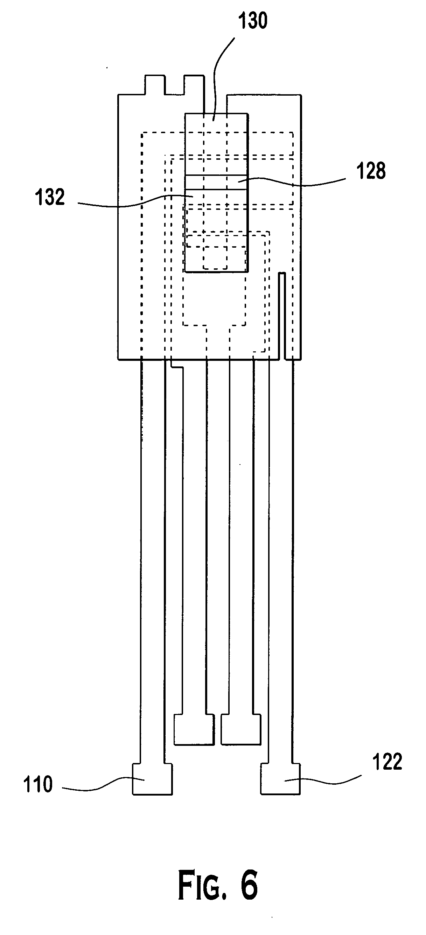

In the embodiments of FIGs. 1-6, the first analyte reagent layer and the second analyte reagent layer are disposed over the shared counter/reference electrode such that the first analyte reagent layer and second analyte reagent layer are separated by a gap over the shared counter/reference electrode (see FIG. 6 in particular). However, the first analyte reagent layer and the second analyte reagent layer can also be disposed over the shared counter/reference electrode such that the first analyte reagent layer and second analyte reagent layer overlap over the shared counter/reference electrode, thus enabling a smaller multi-analyte test strip or the use of beneficially relaxed manufacturing tolerances.

When the first analyte is glucose and the second analyte is the ketone 3-hydroxybutyrate, the mediator layer can contain, for example, potassium ferricyanide and NAD. The first reagent layer can contain glucose oxidase and the second reagent layer can contain diaphorase, and hydroxybutyrate dehydrogenase.

Once apprised of the present invention, one skilled in the art will recognize that the mediator layer, first reagent layer and second reagent layer can also contain suitable buffers, surfactants, thickeners and other additives as are known in the field.

Test meters for use with a multi-analyte test strip according to embodiments of the present invention include a test strip receiving module and a signal processing module. The test strip receiving module has a first electrical connector configured for contacting a first analyte contact pad of a first working electrode of the multi-analyte test strip; a second electrical connector configured for contacting a counter/reference electrode contact pad of a shared counter/reference electrode of the multi-analyte test strip; and a third electrical connector configured for contacting a second analyte contact pad of a second working electrode of the multi-analyte test strip. The signal processing module is configured to receive a first electrical signal via the first electrical connector and the second electrical connector and employ that first signal for the determination of a first analyte (such as glucose) in a bodily fluid sample applied to the multi-analyte test strip. The signal processing module is also configured to receive a second electrical signal via the second electrical connector and third electrical connector and employ the second electrical signal for the determination of a second analyte (such as the ketone 3-hydroxybutyrate) in the bodily fluid sample applied to the multi-analyte test strip. Furthermore, the first analyte electrode, shared counter/reference electrode and second analyte electrode are disposed on a first insulating layer of the multi-analyte test strip in a planar inline configuration. The signal processing module can be configured to receive the first electrical signal and the second electrical signal sequentially, simultaneously or in a time-overlapping manner.

Test meters according to embodiments of the present invention are beneficially small in size, inexpensive and readily manufactured since they employ a single electrical connector for contacting a counter/reference electrode that is used for the determination of multiple analytes. In other words, only three electrical connectors are needed to determine multiple analytes, thus reducing test meter size, cost and manufacturing difficulty. Moreover, the planar configuration of the first analyte electrode, shared counter/reference electrode and second analyte electrode in the multi-analyte test strips employed with the test meter provide for the test meter to be simple in construction by, for example, employing electrical connectors configured in a single plane (i.e., planar electrical connectors).

If desired, test meters according to embodiments of the present invention can be configured to poise a first working electrode and a second working electrode of a multi-analyte test strip at different voltages while still employing a single shared counter/reference electrode. This ability can be particularly beneficial if two mediator layers are employed as described above with respect to multi-analyte test strips according to the present invention. For example, (a) if a first mediator reagent layer containing ferricyanide is employed for the determination of glucose, a poise voltage of 0.4V can, for example, be employed for a first working electrode that is used in the determination of glucose, while (b) if a second mediator reagent layer containing ruthenium hexaminechloride is employed for the determination of 3-hydroxybutyrate at a second working electrode, a poise voltage of 0.25V can be used for the second working electrode.

FIG. 7 is simplified depiction of a test meter 200 according to embodiment of the present invention in use with a conductive layer of an analyte test strip according to an embodiment of the present invention (namely, multi-analyte test strip 100 of FIGs. 1-6). Test meter 200 includes a test strip receiving module 202 and a signal processing module 204 within case 206.

Test strip receiving module 202 includes a first electrical connector 208 configured for contacting first analyte contact pad 110 of a first working electrode 108 of the multi-analyte test strip; a second electrical connector 210 configured for contacting a counter/reference electrode contact pad 114 of a shared counter/reference electrode 112 of the multi-analyte test strip; a third electrical connector 212 configured for contacting a second analyte contact pad 118 of a second working electrode 116 of the multi-analyte test strip; and a fourth electrical connector configured for contacting a fill detect contact pad of a fill detect electrode of the multi-analyte test strip.

Signal processing module 204 is configured to receive a first signal via first electrical connector 208 and second electrical connector 210 and employ the first signal for the determination of a first analyte in a bodily fluid sample applied to the multi-analyte test strip. Signal processing module 204 is also configured to receive a second signal via second electrical connector 210 and third electrical connector 212 and employ the second signal for the determination of a second analyte in the bodily fluid sample applied to the multi-analyte test strip. Moreover, signal processing module 204 is configured to receive a third electrical signal via the fourth electrical contact 214 and employ the third electrical signal to detect that a sample chamber of the multi-analyte test strip has been sufficiently filled by the bodily fluid sample.

In the embodiment of FIG. 7, signal processing module 204 includes, for example, a signal receiving component, a signal measurement component, a processor component and a memory component (each not shown in FIG. 7). Test meter 200 can measure, for example, electrical resistance, electrical continuity or other electrical characteristic between first working electrode 108 and shared counter/reference electrode 112 and between second working electrode 116 and shared counter/reference electrode 112. One skilled in the art will appreciate that the test meter 200 can also employ a variety of sensors and circuits that are not depicted in simplified FIG. 7 during determination of a first analyte and a second analyte.

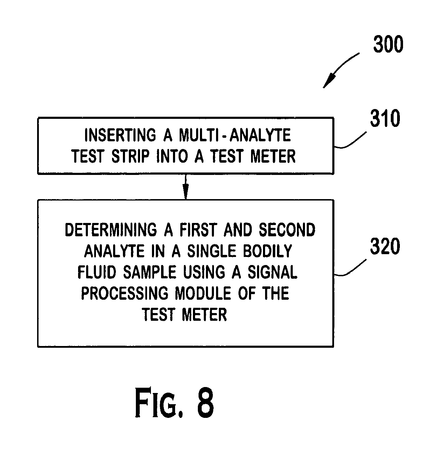

FIG. 8 is a flow diagram depicting stages in a method 300 for determining multiple analytes (for example, the analyte glucose and the analyte 3-hydroxybutyrate) in a single bodily fluid sample (such as a whole blood sample) according to an embodiment of the present invention.

At step 310 of method 300, a multi-analyte test strip is inserted into a test meter. The insertion of the test strip into the meter is such that a first electrical connector of the test meter comes into contact with a first analyte contact pad of a first working electrode of the multi-analyte test strip; a second electrical connector of the test meter comes into contact with a counter/reference electrode contact pad of a shared counter/reference electrode of the multi-analyte test strip; and a third electrical connector of the test meter comes into contact with a second analyte contact pad of a second working electrode of the multi-analyte test strip.

The method also includes determining at least a first analyte and a second analyte in a single bodily fluid sample applied to the multi-analyte test strip using a signal processing module of the test meter (see step 320 of FIG. 8). During the determining step, the signal processing module receives a first signal via the first electrical connector and the second electrical connector and employs the first signal for the determination of a first analyte. Also during the determining step, the signal processing module receives a second signal via the second electrical connector and the third electrical connector and employs the second signal for the determination of a second analyte.

Once apprised of the present disclosure, one skilled in the art will recognize that method 300 can be readily modified to incorporate any of the techniques, benefits and characteristics of multi-analyte test strips according to embodiments of the present invention and described herein, as well as those of test meters according to embodiments of the present invention described herein.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that devices and methods within the scope of these claims and their equivalents be covered thereby.

## Claims

1. A multi-analyte test strip comprising:
a first insulating layer;
an electrically conductive layer disposed on the first insulating layer, the first electrically conductive layer including:
a first working electrode with a first analyte contact pad;
a shared counter/reference electrode with a counter/reference electrode contact pad; and
a second working electrode with a second analyte contact pad a second insulating layer disposed above the first insulating layer;
a patterned spacer layer positioned between the first insulating layer and the first electrically conductive layer, the patterned spacer layer defining a bodily fluid sample-receiving chamber therein that overlies the first working electrode, the shared counter/reference electrode and the second working electrode;
at least one mediator reagent layer disposed over at least a portion of the first working electrode, at least a portion of the shared counter/reference electrode and at least a portion of the second working electrode;
a first analyte reagent layer disposed at least over a portion of the first working electrode; and
a second analyte reagent layer disposed at least over a portion of the second working electrode;
wherein the first analyte electrode, shared counter/reference electrode and second analyte electrode are disposed on the first insulating layer in a planar inline configuration.

2. The multi-analyte test strip of claim 1 wherein the electrically conductive layer further includes a fill detect electrode; and
wherein the sample-receiving chamber also overlies the fill detect electrode; and wherein first analyte electrode portion, shared counter/reference electrode, second analyte electrode portion and fill detect electrode are disposed on the first insulating layer in a planar inline configuration

3. The multi-analyte test strip of claim 1 wherein the first analyte reagent layer and the second analyte reagent layer are also disposed over the shared counter/reference electrode such that the first analyte reagent layer and second analyte reagent layer are separated by a gap over the shared counter/reference electrode or such that the first analyte reagent layer and second analyte reagent layer overlap over the shared counter/reference electrode.

4. The multi-analyte test strip of claim 1 wherein the shared counter/reference electrode is disposed between the first working electrode and the second working electrode in the planar in-line configuration.

5. The multi-analyte test strip of claim 1 wherein the at least one mediator reagent layer includes a first mediator reagent layer and a second mediator reagent layer and wherein the first mediator reagent layer is disposed over at least a portion of the first working electrode and the second mediator reagent layer is disposed over at least a portion of the second working electrode.

6. A test meter for use with a multi-analyte test strip, the test meter comprising:
a test strip receiving module with at least:
a first electrical connector configured for contacting a first analyte contact pad of a first working electrode of the multi-analyte test strip;
a second electrical connector configured for contacting a counter/reference electrode contact pad of a shared counter/reference electrode of the multi-analyte test strip; and
a third electrical connector configured for contacting a second analyte contact pad of a second working electrode of the multi-analyte test strip; and
a signal processing module,
wherein the signal processing module is configured to receive a first signal via the first electrical connector and the second electrical connector and employ the first signal for the determination of a first analyte in a bodily fluid sample applied to the multi-analyte test strip; and
wherein the signal processing module is also configured to receive a second signal via the second electrical connector and third electrical connector and employ the second signal for the determination of a second analyte in the bodily fluid sample applied to the multi-analyte test strip; and
wherein the first analyte electrode, shared counter/reference electrode and second analyte electrode are disposed on a first insulating layer of the multi-analyte test strip in a planar inline configuration.

7. The test meter of claim 6 wherein the test strip receiving module further includes a fourth electrical connector configured for contacting a fill detect contact pad of a fill detect electrode of the multi-analyte test strip; and
wherein the signal processing module is configured to receive a third electrical signal via the fourth electrical contact and employ the third electrical signal to detect that a sample chamber of the multi-analyte test strip has been sufficiently filled by the bodily fluid sample.

8. A method for determining multiple analytes in a single bodily fluid sample, the method comprising:
inserting a multi-analyte test strip into a test meter such that:
a first electrical connector of the test meter comes into contact with a first analyte contact pad of a first working electrode of the multi-analyte test strip;
a second electrical connector of the test meter comes into contact with a counter/reference electrode contact pad of a shared counter/reference electrode of the multi-analyte test strip; and
a third electrical connector of the test meter comes into contact with a second analyte contact pad of a second working electrode of the multi-analyte test strip;
determining a first analyte and a second analyte in a single bodily fluid sample applied to the multi-analyte test strip using a signal processing module of the test meter,
wherein, during the determining step, the signal processing module receives a first signal via the first electrical connector and the second electrical connector and employs the first signal for the determination of a first analyte; and
wherein, during the determining step, the signal processing module receives a second signal via the second electrical connector and the third electrical connector and employs the second signal for the determination of a second analyte; and
wherein the first analyte electrode, shared counter/reference electrode and second analyte electrode are disposed on a first insulating layer of the multi-analyte test strip in a planar inline configuration.

9. The method of claim 8 wherein the inserting step further includes inserting the multi-analyte test strip such that a fourth electrical connector of the test meter contacts a fill detect contact pad of a fill detect electrode of the multi-analyte test strip; and
wherein, during the determining step, the signal processing module receives a third electrical signal via the fourth electrical contact and employs the third electrical signal to detect that a sample chamber of the multi-analyte test strip has been sufficiently filled by the single bodily fluid sample.

10. The multi-analyte test strip of claim 1 or the test meter of claim 6 or the method of claim 8 wherein the first analyte is nonidentical in comparison to the second analyte.

11. The multi-analyte test strip or the test meter or the method of claim 10 wherein the first analyte is glucose.

12. The multi-analyte test strip or the test meter or the method of claim 11 wherein the second analyte is a ketone.

13. The multi-analyte test strip or the test meter or the method of claim 12 wherein the ketone is 3-hydroxybutyrate.

14. The multi-analyte test strip of claim 1 or the test meter of claim 6 or the method of claim 8 wherein the bodily fluid sample is a whole blood sample.

15. The test meter of claim 6 or the method of claim 8 wherein the signal processing module is configured for determination of the first analyte and determination of the second analyte via an electrochemical-based determination technique.

16. The test meter of claim 6 or the method of claim 8 wherein the signal processing module is configured to receive the first signal and the second signal in a sequential manner or in a simultaneous manner.
